(19) 

Europäisches
Patentamt
European
Patent Office
Office européen
des brevets

(11) **EP 4 617 369 A1**

(12) **EUROPEAN PATENT APPLICATION**
published in accordance with Art. 153(4) EPC

(43) Date of publication:
**17.09.2025 Bulletin 2025/38**

(21) Application number: **25727638.6**

(22) Date of filing: **31.01.2025**

(51) International Patent Classification (IPC):
*C12P 13/08* *(2006.01)*

(86) International application number:
**PCT/KR2025/001580**

(87) International publication number:
**WO 2025/165149 (07.08.2025 Gazette 2025/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **01.02.2024 KR 20240016196**

(71) Applicant: **CJ Cheiljedang Corporation
Seoul 04560 (KR)**

(72) Inventors:
• **KWAK, Dong Hun
  Seoul 04560 (KR)**

• **PARK, Sang Min
  Seoul 04560 (KR)**
• **PARK, Jeong Ho
  Seoul 04560 (KR)**
• **SHIN, Jihyun
  Seoul 04560 (KR)**
• **LEE, Su Jin
  Seoul 04560 (KR)**
• **LEE, Yong-Chan
  Seoul 04560 (KR)**

(74) Representative: **Meissner Bolte Partnerschaft
mbB
Patentanwälte Rechtsanwälte
Postfach 86 06 24
81633 München (DE)**

(54) **METHOD FOR PRODUCING HIGH-CONTENT LYSINE USING AUTOMATED GAS
INTRODUCTION SYSTEM**

(57)     The present disclosure relates to a method for preparing lysine, which monitors ammonium nitrogen in a medium and pH in real time, and according to that, carbon dioxide and/or nitrogen sources are introduced, thereby significantly improving the concentration of lysine while reducing the concentration of by-products, and improving the fermentation purity and enhancing the quality of lysine granules.

【FIG. 3】

**Description**

[TECHNICAL FIELD]

Cross-reference to prior application(s)

The present disclosure claims the benefit of the priority based on Korean Patent Application No. 10-2024-0016196 filed on February 1, 2024, and the entire contents disclosed in the document of the corresponding Korean patent application are incorporated as a part of the present disclosure.

Throughout the present disclosure, numerous papers and patent documents are referenced and citations thereof are indicated. The disclosures of the cited papers and patent documents are incorporated by reference into the present disclosure in their entirety to more clearly describe the level of the technical field to which the present disclosure pertains and the contents of the present disclosure.

The present disclosure relates to a method for preparing lysine, and more specifically, relates to a method for preparing lysine at a high content using a gas automated introduction system.

[BACKGROUND ART]

Lysine (L-lysine) is one of essential amino acids, and is used in various fields, mainly, such as feed additives, food additives, raw materials of pharmaceuticals, and is one of bulk chemicals with a market size at a level of 2.94 million tons as of 2022. Currently, most of lysine is mass-produced by direct fermentation using a medium containing a carbon source (raw sugar, sucrose, glucose) and a nitrogen source (yeast extract, soybean meal hydrolysate, corn steep liquor).

In ammonium sulfate added among medium components supplied for lysine fermentation using a Corynebacterium-shaped microorganism, sulfate performs a role as a counter ion to maintain a neutral pH condition for lysine biosynthesized during fermentation, and ammonium performs a role of supplying a nitrogen source required for lysine biosynthesis. Consequently, in order to be present in the form of lysine sulfate in the culture solution and commercialize it in the form of lysine hydrochloride (Lysine HCl), which is a representative powder-type formulation component, a process of adsorbing and eluting lysine by an ion exchange resin process is necessary. However, since addition of a purification process and addition of subsidiary raw materials are necessary in this process, it becomes a factor that significantly increases productions costs.

In order to overcome the above problems, there is a method for fermentation that removes ammonium sulfate added during fermentation (Chinese Patent Publication No. 110484575). The corresponding research relates to a method for preparing lysine comprising carbonates, and carbonates are produced during fermentation or supplied through external carbonate aqueous solution and carbon dioxide gas. Production costs for the fermented solution obtained by the corresponding method can be significantly reduced by introduction of granulation formulation (Korean Patent No. 10-0838200). With the advantages of low hygroscopicity and great flowability of granular products, through formulation diversification, the problems of the process can be overcome and cost competitiveness can be significantly improved. However, other by-products except for lysine produced during fermentation is a major cause to lower the purity of the products, when granulation is conducted without ion exchange resin treatment in the process of preparing granules using lysine carbonate fermentation, so it is necessary to reduce organic acid and amino acid by-products and improve the yield of products.

[DISCLOSURE]

[TECHNICAL PROBLEM]

An object of the present disclosure is to provide a method of preparing lysine at a high content using a gas automated introduction system.

The present inventors of the present disclosure have confirmed that the concentration of lysine is significantly improved while the concentration of by-products is reduced, thereby improving the fermentation purity and enhancing the quality of lysine granules, by monitoring the concentration of ammonium nitrogen (hereinafter, also referred to as "AN") in a medium, in a fermentation process using a microorganism having an ability to produce lysine, and when the pH condition reaches a specific range and the ammonium nitrogen is reduced to a specific numerical value or less, a nitrogen source and/or carbon dioxide is introduced into the medium to control the ammonium nitrogen in an appropriate range, thereby completing the invention of the method for preparing lysine of the present disclosure.

[TECHNICAL SOLUTION]

This is explained in detail as follows. On the other hand, each description and embodiment disclosed in the present application can also be applied to each other description and embodiment. In other words, all combinations of the various elements disclosed in the present application belong to the scope of the present application. In addition, the scope of the present application cannot be considered limited by the specific description described below. In addition, throughout the present description, numerous papers and patent documents are referenced and citations thereof are indicated. The disclosures of the cited papers and patent documents are incorporated in the present description by reference in their entirety to more clearly describe the level of the technical field to which the present disclosure pertains and the contents of the present disclosure.

According to one aspect of the present disclosure, the present disclosure provides a method for preparing lysine by a fermentation process using a microorganism having an ability to produce lysine, characterized by

monitoring the concentration of ammonium nitrogen in a medium, and
introducing a nitrogen source into the medium.

The inventors of the present disclosure have confirmed that the concentration of lysine is significantly improved while the concentration of by-products is reduced, thereby improving the fermentation purity and enhancing the quality of lysine granules, by monitoring the concentration of ammonium nitrogen (hereinafter, also referred to as "AN") in a medium, in a method for preparing lysine by a fermentation process using a microorganism having an ability to produce lysine, and when the pH condition reaches a specific range and the ammonium nitrogen is reduced to a specific numerical value or less, a nitrogen source and/or carbon dioxide is introduced into the medium to control the ammonium nitrogen in an appropriate range, thereby completing the invention of the method for preparing lysine of the present disclosure.

In the present disclosure, the nitrogen source and/or carbon dioxide may be automatically introduced into a medium. More specifically, the nitrogen source and/or carbon dioxide may be automatically introduced into a medium through a gas automated introduction system.

In the present disclosure, "gas automated introduction system" refers to monitoring the concentration of ammonium nitrogen in the medium and/or the pH of the medium during the fermentation process using a microorganism having an ability to produce lysine in real time, and automatically introducing ammonia gas and/or ammonia solution as nitrogen sources under a specific condition, and/or automatically introducing carbon dioxide into the medium to adjust the ammonium nitrogen and/or pH in an appropriate range. When such a gas automated introduction system is used, the concentration of by-products during culture is reduced and also the concentration of lysine is significantly improved and therefore, the fermentation purity can be improved and the quality of lysine granules can be enhanced.

The microorganism having an ability to produce lysine used in the method for preparing lysine of the present disclosure is not particularly limited, and as long as they can produce lysine by fermentation, all microorganisms can be used. The examples of such a microorganism include Corynebacterium-shaped bacteria and bacteria belonging to the genus of Escherichia, Serratia, Bacillus and the like. The Corynebacterium-shaped bacteria and bacteria belonging to the genus of Escherichia are described below, but the microorganism used in the method of the present disclosure is not limited to these bacteria.

The Corynebacterium-shaped bacteria (or hereinafter, also referred to as "Corynebacterium-shaped microorganism" ) are integrated into the genus of Corynebacterium, and include bacteria belonging to the genus of Brevibacterium closely related to the genus of Corynebacterium. The examples of the Corynebacterium-shaped bacteria are mentioned below:

*Corynebacterium acetoacidophilum,*
*Corynebacterium acetoglutamicum,*
*Corynebacterium alkanolyticum,*
*Corynebacterium callunae,*
*Corynebacterium glutamicum,*
*Corynebacterium lilium (Corynebacterium glutamicum),*
*Corynebacterium melassecola,*
*Corynebacterium thermoaminogenes,*
*Corynebacterium herculis,*
*Brevibacterium divaricatum,*
*Brevibacterium flavum (Corynebacterium glutamicum),*
*Brevibacterium immariophilum,*
*Brevibacterium lactofermentum (Corynebacterium glutamicum),*
*Brevibacterium roseum,*

*Brevibacterium saccharolyticum,*
*Brevibacterium thiogenitalis,*
*Brevibacterium album,*
*Brevibacterium cerinum,*
*Microbacterium ammoniaphilum.*

As the example of the bacteria belonging to the genus of Escherichia, Escherichia coli may be included, but not limited thereto.

The microorganism having an ability to produce lysine may naturally have an ability to produce lysine, or be modified to have an ability produce lysine. The microorganism having an ability to produce lysine may be obtained by giving an ability to produce lysine into the microorganism as above, or enhancing an ability to produce lysine of the microorganism as above.

In one embodiment, the microorganism having an ability to produce lysine may be *Corynebacterium glutamicum,* and more specifically, it may be *Corynebacterium glutamicum* CJ3P having an ability to produce L-lysine by introducing conventionally known mutations of pyc(P458S), hom(V59A), lysC(T311I) into three kinds of genes using *Corynebacterium glutamicum* ATCC13032 as a parent strain (US 9556463 B2), but not limited thereto.

In the present disclosure, the culture method is not limited, but it may be performed by for example, batch culture, fed-batch culture or continuous culture. In one specific example, in the present disclosure, the seed culture was performed by batch culture, and the main culture was performed by batch culture and then fed-batch culture, but not limited thereto.

The medium used in the present disclosure may be a liquid medium, and this liquid medium is not particularly limited, and is all commonly known media containing organic or inorganic nutrients such as a carbon source and a nitrogen source and other trace nutrients that can be used according to the microorganism used.

As long as the microorganism can use them, all carbon sources may be used. For example, sugars such as saccharose, glucose, fructose, molasses, and starch hydrolysates, organic acids such as acetate, alcohols such as ethanol and the like may be mentioned. As a nitrogen source, inorganic substances such as ammonium ion, protein hydrolysates, yeast extracts and the like may be mentioned. As a trace nutrient, amino acids, vitamins, trace metal elements and the like may be mentioned.

In addition, the pH of the medium may be adjusted using basic compounds such as sodium hydroxide, potassium hydroxide and ammonia, or acid compounds such as phosphoric acid or sulfuric acid in an appropriate manner. Furthermore, bubble generation may be inhibited using an antifoaming agent such as fatty acid polyglycol ester.

On the other hand, the culture temperature that can be suitably selected depending on the microorganism used may be generally, 20 to 45°C, preferably, 25 to 40°C. In addition, sufficient stirring is performed during fermentation, and sufficient oxygen is supplied. In order to maintain an aerobic state, oxygen or oxygen-containing gas (e.g., air) is introduced into culture.

In one embodiment, in order to use as a nitrogen source and a counter anion source in the medium, ammonium sulfate may be further comprised. More specifically, the ammonium sulfate may be comprised in the medium at the beginning of the culture, more specifically, at the start time of the culture. In the ammonium sulfate, sulfate performs a role as a counter ion for lysine, and ammonium performs a role of supplying a nitrogen source required for lysine biosynthesis. In the present disclosure, it is characterized by using such ammonium sulfate at the least. Specifically, ammonium sulfate used in the present disclosure may be comprised by 0.2 (mol/mol) or more, 0.3 (mol/mol) or more, 0.4 (mol/mol) or more, 0.45 (mol/mol) or more, 1.0 (mol/mol) or less, 0.8 (mol/mol) or less, 0.7 (mol/mol) or less, 0.6 (mol/mol) or less, 0.5 (mol/mol) or less, 0.2 (mol/mol) to 1.0 (mol/mol), 0.3 (mol/mol) to 1.0 (mol/mol), 0.4 to 1.0 (mol/mol), 0.45 (mol/mol) to 1.0 (mol/mol), 0.46 (mol/mol) to 1.0 (mol/mol), 0.2 (mol/mol) to 0.8(mol/mol), 0.3 (mol/mol) to 0.8 (mol/mol), 0.4 to 0.8 (mol/mol), 0.45 (mol/mol) to 0.8 (mol/mol), 0.46 (mol/mol) to 0.8 (mol/mol), 0.2 (mol/mol) to 0.7 (mol/mol), 0.3 (mol/mol) to 0.7 (mol/mol), 0.4 (mol/mol) to 0.7 (mol/mol), 0.45 (mol/mol) to 0.7 (mol/mol), 0.46 (mol/mol) to 0.7 (mol/mol), 0.2 (mol/mol) to 0.6 (mol/mol), 0.3 (mol/mol) to 0.6 (mol/mol), 0.4 (mol/mol) to 0.6 (mol/mol), 0.45 (mol/mol) to 0.6 (mol/mol), 0.46 (mol/mol) to 0.6 (mol/mol), 0.2 (mol/mol) to 0.5 (mol/mol), 0.3 (mol/mol) to 0.5 (mol/mol), 0.4 (mol/mol) to 0.5 (mol/mol), 0.45 (mol/mol) to 0.5 (mol/mol), or 0.46 (mol/mol) to 0.5 (mol/mol) at a molar ratio of ammonium sulfate to the moles of the carbon source, but not limited thereto. Therefore, when the method for preparing lysine of the present disclosure is used, a high cost of process of adsorbing and eluting lysine by an ion exchange resin process that is essentially used when a large amount of ammonium sulfate is used is not used.

Specifically, in the present disclosure, in order to slightly increase the concentration of ammonium nitrogen in the medium at the beginning of culture, ammonium sulfate is comprised in the medium at the initial step of the culture (more specifically, at the time of the start of the culture), and after that, during the fermentation process (during culture), ammonium sulfate is not further added into the medium.

In one embodiment, monitoring the concentration of ammonium nitrogen in the medium may be performed using a near infrared (NIR) spectrometric analyzer.

The NIR can continuously analyze various kinds of components simultaneously in a short time without pretreatment of samples, so can analyze methods of analysis by each component by the conventional wet analysis method, that is, the solid liquid chromatography (HPLC) which analyzes sugars, amino acids, nucleic acids, and the like, or a Kjeldahl analysis method for nitrogen component analysis at the same time at once without pretreatment of samples, and thus, real-time analysis becomes possible by directly attaching a sensor or probe to a fermented solution. Therefore, it is a technology spotlighted as a new real-time quantitative analysis method which is a method that has been attempted to be applied to various processes as a method which can not only significantly reduce the analysis time, costs, and labor and the like, but also make rapid quantitative analysis in seconds or tens of seconds possible without the use of hazardous chemicals used for pretreatment and the like.

By this NIR, in the present disclosure, unlike the conventional Kjeldahl analysis method, which is cumbersome and time-consuming to analyze a portion of the fermented solution obtained by sampling during culture, the concentration of ammonium nitrogen (AN) in the medium can be analyzed in real time online.

Therefore, in a specific embodiment, the concentration of ammonium nitrogen in the medium can be monitored in real time, and monitoring the concentration of ammonium nitrogen in real time may be performed using a near infrared spectrometric analyzer.

As demonstrated in the example to be described later, the inventors of the present disclosure have established a model that can monitor the concentration of ammonium nitrogen (AN) in real time with high accuracy using NIR spectroscopy.

In one embodiment, in the method for preparing lysine of the present disclosure described above, the pH of the medium may be further monitored, and more specifically, the pH may be monitored in real time.

In one embodiment, the pH of the medium may be measured and recorded in real time using a pH meter.

In one embodiment, in the method for preparing lysine of the present disclosure described above, when the concentration of ammonium nitrogen in the medium is less than 1.5 g/kg, the nitrogen source may be introduced into the medium.

In the present disclosure, when the concentration of ammonium nitrogen in the medium is less than 1.5 g/kg, it may mean the case in that the concentration of ammonium nitrogen in the medium is less than 1.5 g/kg, less than 1.45 g/kg, less than 1.4 g/kg, less than 1.35 g/kg, less than 1.3 g/kg, less than 1.25 g/kg, less than 1.2 g/kg, less than 1.15 g/kg, less than 1.1 g/kg, less than 1.05 g/kg or less than 1.0 g/kg, but not limited thereto, and decimals less than 1.5 g/kg are included in the range of the present disclosure.

In one embodiment, the nitrogen source may be at least one selected from the group consisting of ammonia gas and ammonia solution.

In one embodiment, in the method for preparing lysine of the present disclosure described above, the concentration of ammonium nitrogen in the medium may be controlled to be maintained at 1.0 g/kg to 3.0 g/kg, and more specifically, the concentration of ammonium nitrogen in the medium may be controlled to be maintained at 1.0 g/kg to 3.0 g/kg, 1.0 g/kg to 2.5 g/kg, 1.0 g/kg to 2.0 g/kg, 1.0 g/kg to 1.75 g/kg, 1.0 g/kg to 1.5 g/kg, 1.0 g/kg to 1.25 g/kg, 1.25 g/kg to 3.0 g/kg, 1.25 g/kg to 2.5 g/kg, 1.25 g/kg to 2.0 g/kg, 1.25 g/kg to 1.75 g/kg, 1.25 g/kg to 1.5 g/kg, 1.5 g/kg to 3.0 g/kg, 1.5 g/kg to 2.5 g/kg, 1.5 g/kg to 2.0 g/kg, or 1.5 g/kg to 1.75 g/kg but not limited thereto. More specifically, as described above, when the concentration of ammonium nitrogen in the medium is less than 1.5 g/kg, by adding the nitrogen source into the medium, the concentration of ammonium nitrogen in the medium may be adjusted to be maintained at 1.0 g/kg to 3.0 g/kg, 1.0 g/kg to 2.5 g/kg, 1.0 g/kg to 2.0 g/kg, 1.0 g/kg to 1.75 g/kg, 1.0 g/kg to 1.5 g/kg, 1.0 g/kg to 1.25 g/kg, 1.25 g/kg to 3.0 g/kg, 1.25 g/kg to 2.5 g/kg, 1.25 g/kg to 2.0 g/kg, 1.25 g/kg to 1.75 g/kg, 1.25 g/kg to 1.5 g/kg, 1.5 g/kg to 3.0 g/kg, 1.5 g/kg to 2.5 g/kg, 1.5 g/kg to 2.0 g/kg, or 1.5 g/kg to 1.75 g/kg. By controlling the concentration of ammonium nitrogen in the medium to be maintained at 1.0 g/kg to 3.0 g/kg, the metabolic activity of the microorganism having an ability to produce lysine may be maintained to enhance the fermentation rate and increase the fermentation yield.

In the present disclosure, introducing the nitrogen source into the medium may involve introducing the nitrogen source automatically. Specifically, the nitrogen source may be automatically introduced by the HMI (Human-Machine Interface) automated program, but not limited thereto.

In one embodiment, introducing the nitrogen source into the medium may involve increasing the pH set-point of the medium and then introducing the nitrogen source into the medium so that the pH of the medium can reach the pH set-point.

Increasing the pH set-point may be controlled by the HMI (Human-Machine Interface) automated program, but not limited thereto.

In one embodiment, when the concentration of ammonium nitrogen in the medium is less than 1.5 g/kg, the pH set-point may be slightly increased. More specifically, the pH set-point may be increases as much as approximately 0.05, for example, as much as 0.01 to 0.1, 0.01 to 0.075, 0.01 to 0.06, 0.01 to 0.05, 0.025 to 0.1, 0.025 to 0.075, 0.025 to 0.06, 0.025 to 0.05, 0.03 to 0.1, 0.03 to 0.075, 0.03 to 0.06, 0.03 to 0.05, 0.04 to 0.1, 0.04 to 0.075, 0.04 to 0.06, or 0.04 to 0.05.

More specifically, when the concentration of ammonium nitrogen in the medium is less than 1.5 g/kg, the pH set-point may be upregulated to about 0.05 by the HMI (Human-Machine Interface) automated program, and the concentration of ammonium nitrogen in the medium may be increased, by introducing the nitrogen source so that the pH of the medium can reach the pH set-point.

In one embodiment, the pH of the medium may be further monitored, and when the pH of the medium is pH 7.5 or more and less than pH 8.0 and the concentration of ammonium nitrogen in the medium is less than 1.5 g/kg, the pH set-point may be increased, and so as to reach the pH set-point, the nitrogen source may be introduced.

In the present disclosure, when the pH of the medium is pH 7.5 or more and less than pH 8.0, it may refer to a case when the pH of the medium is pH 7.5 or more and less than pH 8.0, pH 7.6 or more and less than pH 8.0, pH 7.7 or more and less than pH 8.0, pH 7.8 or more and less than pH 8.0, pH 7.9 or more and less than pH 8.0, pH 7.5 or more and less than pH 7.9, pH 7.6 or more and less than pH 7.9, pH 7.7 or more and less than pH 7.9, or pH 7.8 or more and less than pH 7.9, but not limited thereto.

In the present disclosure, when the concentration of ammonium nitrogen is less than 1.5 g/kg as above, as the pH of the medium is slightly increased gradually by adding the nitrogen source, the pH of the medium is increased from pH 7.5 or more and less than pH 8.0 to pH 8.0 or more and less than pH 8.5.

In the present disclosure, when the pH of the medium is pH 8.0 or more and less than pH 8.5, it may refer to a case in that the pH of the medium is pH 8.0 or more and less than pH 8.5, pH 8.0 or more and less than pH 8.4, pH 8.0 or more and less than pH 8.3, pH 8.0 or more and less than pH 8.2 or pH 8.0 or more and less than pH 8.1, but not limited thereto.

In one embodiment, the pH of the medium may be further monitored, and when the pH of the medium is pH 8.0 or more and less than pH 8.5 and the concentration of ammonium nitrogen in the medium is less than 1.5 g/kg, carbon dioxide may be introduced into the medium.

In the present disclosure, the introducing carbon dioxide into the medium may involve automatic introduction carbon dioxide. Specifically, the carbon dioxide may be automatically introduced by the HMI (Human-Machine Interface) automated program, but not limited thereto.

As described above, when the pH of the medium is increased and becomes pH 8.0 or more and less than pH 8.5, since the growth and metabolism of the microorganism having an ability to produce lysine are inhibited due to the increased pH, the fermentation rate and yield may be reduced, and by-products may be generated. Accordingly, when the pH of the medium is pH 8.0 or more and less than pH 8.5 and the concentration of ammonium nitrogen in the medium is less than 1.5 g/kg, carbon dioxide may be introduced into the medium to decrease the pH of the medium.

In one embodiment, the carbon dioxide may be introduced until the pH of the medium decreases by 0.1 to 0.4, more specifically, by 0.1 to 0.4, 0.1 to 0.3, 0.1 to 0.2, 0.2 to 0.4, or 0.2 to 0.3, but not limited thereto.

In one embodiment, the carbon dioxide may be introduced at 0.5 vvm to 3.0 vvm. More specifically, the amount of airflow at the time of introducing carbon dioxide may be adjusted suitably by those skilled in the art, and for example, it may be introduced at 0.5 to 3.0 vvm, more specifically, 0.5 to 3.0 vvm, 0.5 to 2.0 vvm, 0.5 to 1.7 vvm, 0.5 to 1.5 vvm, 0.7 to 3.0 vvm, 0.7 to 3.0 vvm, 0.7 to 2.0 vvm, 0.7 to 1.7 vvm, 0.7 to 1.5 vvm, 1.0 to 3.0 vvm, 1.0 to 2.0 vvm, 1.0 to 1.7 vvm, or 1.0 to 1.5 vvm (gas introduction amount L / medium volume L / minute), but not limited thereto.

In one embodiment, the nitrogen source may be introduced into the medium so that the pH of the medium decreased by the introduction of carbon dioxide can be increased. More specifically, the nitrogen source may be introduced into the medium so that the pH of the medium decreased by the introduction of carbon dioxide can again reach the pH set-point previously set. The pH set-point previously set may be pH 8.0 or more and less than pH 8.5. The nitrogen source may be introduced simultaneously or sequentially with introducing the carbon dioxide into the medium. The method for introducing the nitrogen source and the like are as described above.

In one embodiment, the process of introducing carbon dioxide into the medium and introducing the nitrogen source simultaneously or sequentially when the pH of the medium is pH 8.0 or more and less than pH 8.5 and the concentration of ammonium nitrogen in the medium is less than 1.5 g/kg may be repeatedly performed. More specifically, in case that the pH of the medium is pH 8.0 or more and less than pH 8.5 and the concentration of ammonium nitrogen in the medium is less than 1.5 g/kg, introducing carbon dioxide into the medium decreases the pH of the medium. When the nitrogen source is introduced into the medium so that the decreased pH of the medium can reach the pH set-point set previously, the pH increases again and reaches the range of pH 8.0 or more and less than pH 8.5. In this manner, when the pH reaches pH 8.0 or more and less than pH 8.5, and as fermentation progresses, the concentration of ammonium nitrogen in the medium becomes less than 1.5 g/kg again, the above process can be repeatedly performed. As the above process is repeatedly performed, the concentration of ammonium nitrogen in the medium may be maintained in the range of 1.0 g/kg or more, specifically, 1.0 g/kg to 3.0 g/kg, and the pH of the medium may be controlled to less than pH 8.5, specifically, pH 7.5 or more and less than pH 8.5.

In one embodiment, the pH of the medium may be increased from pH 6.5 or more and less than pH 7.0 to pH 7.5 or more and less than pH 8.5, and then carbon dioxide may be introduced to control the pH of the medium to be maintained at pH 7.5 or more and less than pH 8.5.

In a specific embodiment, before increasing the pH from pH 6.5 or more and less than pH 7.0 to pH 7.5 or more and less than pH 8.5 as above, the pH of the medium may be maintained at the value within the range of pH 6.5 or more and less than pH 7.0.

Specifically, at the beginning of fermentation, the pH of the medium may be maintained at the value of pH 6.5 or more and less than pH 7.0. Without separate control, the pH at the beginning of fermentation is gradually decreased due to organic acid accumulation and carbon dioxide gas discharge according to sugar exhaustion. Therefore, to maintain the optimal pH for fermentation, the pH may be maintained at pH 6.5 or more and less than pH 7.0 by introducing ammonia gas and/or ammonia solution, and more specifically, it may be maintained at pH 6.5, pH 6.6, pH 6.7, pH 6.8, or pH 6.9, but not limited thereto, and all decimals comprised in pH 6.5 or more and less than pH 7.0 are included in the range of the present disclosure. Maintaining the pH of the medium at the value of pH 6.5 or more and less than pH 7.0 may be setting the set-point of the pH to the numerical value in the range of pH 6.5 or more and less than pH 7.0, and introducing a nitrogen source, more specifically, ammonia gas and/or ammonia solution by the HMI (Human-Machine Interface) automated program to maintain the pH of the medium at the pH set-point, but not limited thereto. The 'beginning of fermentation' may refer to the period from the start of fermentation until depletion of the carbon source in the initial fermentation medium, but not limited thereto.

In a specific embodiment, when the carbon source in the initial fermentation medium is depleted, a feeding medium may be introduced and the pH of the medium may be increased to pH 6.5 or more and less than pH 7.0 to pH 7.5 or more and less than pH 8.0. The increasing of the pH may be for increasing supply of the nitrogen source after adding the feeding medium. Increasing the pH as above may be increasing the set-point of the pH at a rate of 0.2 to 0.8 per hour, and more specifically, increasing it at a rate of 0.2 to 0.8, 0.2 to 0.8, 0.2 to 0.6, 0.2 to 0.5, 0.3 to 0.8, 0.3 to 0.8, 0.3 to 0.6, 0.3 to 0.5, 0.4 to 0.8, 0.4 to 0.8, 0.4 to 0.6, or 0.4 to 0.5 per hours. Specifically, the pH of the medium may be increased at a constant rate. Specifically, increasing the pH may be introducing a nitrogen source, more specifically, ammonia gas and/or ammonia solution by the HMI (Human-Machine Interface) automated program to allow the pH of the medium to reach the pH set-point, but not limited thereto.

When the pH of the medium reaches pH 7.5 or more and less than pH 8.0 through the above process, the process of introducing the nitrogen source into the medium when the concentration of ammonium nitrogen in the medium described above becomes less than 1.5 g/kg, which is described above, may be performed. Through the above process, the pH of the medium may be increased to pH 8.0 or more and less than pH 8.5. When the pH of the medium reaches pH 8.0 or more and less than pH 8.5, the process of introducing carbon dioxide and introducing the nitrogen source simultaneously or sequentially thereto when the concentration of ammonium nitrogen in the medium becomes less than 1.5 g/kg, which is described above, may be performed, and the pH of the medium may be controlled to be maintained at pH 7.5 or more and less than pH 8.5.

In this manner, the method for preparing lysine of the present disclosure may include the following steps:

> (a) increasing the pH of the medium from pH 6.5 or more and less than pH 7.0 to pH 7.5 or more and less than pH 8.0;
> (b) increasing the pH set-point and introducing the nitrogen source into the medium so that the pH of the medium can reach the pH set-point, when the pH of the medium is pH 7.5 or more and less than pH 8,0, and the concentration of ammonium nitrogen in the medium is less than 1.5 g/kg;
> (c) introducing carbon dioxide gas to decrease the pH and introducing the nitrogen source, when the pH reaches pH 8.0 or more and less than pH 8.5.

In a specific embodiment, prior to step (a), the method for preparing lysine of the present disclosure may comprise maintaining the pH of the medium at a value within a range of pH 6.5 or more and less than pH 7.0. In this case, the method for maintaining the pH value of the medium and the like are as described above.

In the step (a) and step (b), the contents such as the method for increasing the pH and the like are as described above, respectively.

In the step (c), the contents such as the method for reducing the pH, the method for introducing the nitrogen source, and the like are as described above.

In a specific embodiment, the process of the step (c) may be repeatedly performed. Specifically, when the pH of the medium is pH 8.0 or more and less than pH 8.5, and the concentration of ammonium nitrogen in the medium is less than 1.5 g/kg, the automatic introduction of carbon dioxide into the medium decreases the pH of the medium. When the nitrogen source is automatically introduced into the medium so that the pH of the medium decreased by the introduction of carbon dioxide can again reach the pH set-point previously set, the pH is increased again and becomes pH 8.0 or more and less than pH 8.5. In this manner, when the pH reaches pH 8.0 or more and less than pH 8.5, and as fermentation progresses, the concentration of ammonium nitrogen in the medium becomes less than 1.5 g/kg again, the above process can be repeatedly performed. As the above process is repeatedly performed, the concentration of ammonium nitrogen in the medium may be maintained in the range of 1.0 g/kg or more, specifically, 1.0 g/kg to 3.0 g/kg, and the pH of the medium may be controlled to less than pH 8.5, specifically, pH 7.5 or more and less than pH 8.5.

As such, according to the present disclosure, the concentration of ammonium nitrogen in the medium and the pH of the medium are managed in the optimal level range for lysine biosynthesis, so through reduction in fermentation by-products and yield improvement, the purity of the process solution may be improved, thereby significantly reducing production costs.

In the present disclosure, "culture" refers to a process of proliferating a specific microorganism or cell under a condition in which a suitable environment (nutrients, temperature, pH, oxygen concentration, etc.) is provided, or inducing it to produce a desired metabolite, and according to the purpose of the present disclosure, it may be interchangeably used with 'fermentation'.

In the present disclosure, the culture is continued until the produced amount of lysine is obtained at maximum. For this purpose, it may be performed commonly for 10 hours to 160 hours, more specifically, for 10 hours to 160 hours, 10 hours to 100 hours, 10 hours to 50 hours, 10 hours to 35 hours, 20 hours to 160 hours, 20 hours to 100 hours, 20 hours to 50 hours, 20 hours to 35 hours, 30 hours to 160 hours, 30 hours to 100 hours, 30 hours to 50 hours, or 30 hours to 35 hours, but not limited thereto. Lysine may be released into the culture medium, or be comprised in the cell.

In the case of the method for preparing lysine of the present disclosure, compared to the case where culture is performed while controlling the concentration of ammonium nitrogen in the medium to be maintained at 1.0 g/kg to 3.0 g/kg through introduction of the nitrogen source as in the present disclosure, but without the carbon dioxide introduction process of the present disclosure, the by-products may be reduced to the level of about 30% to about 80%, and more specifically, may be reduced to the level of about 30% to about 80%, about 30% to about 75%, about 30% to about 72%, about 30% to about 71.5%, about 45% to about 80%, about 45% to about 75%, about 45% to about 72%, about 45% to about 71.5%, about 60% to about 80%, about 60% to about 75%, about 60% to about 72%, about 60% to about 71.5%, about 65% to about 80%, about 65% to about 75%, about 65% to about 72%, about 65% to about 71.5%, about 70% to about 80%, about 70% to about 75%, about 70% to about 72%, or about 70% to about 71.5%, but not limited thereto.

In the case of the method for preparing lysine of the present disclosure, compared to the case where the culture is performed by introducing carbon dioxide as in the present disclosure and introducing a nitrogen source according to the present disclosure, but introducing the nitrogen source when the concentration of ammonium nitrogen in the medium becomes less than 0.3 g/kg rather than it becomes less than 1.5 g/kg, the by-products may be reduced to the level of about 1% to about 30%, and more specifically, may be reduced to the level of about 1% to about 30%, about 1% to about 20%, about 1% to about 17.5%, about 5% to about 30%, about 5% to about 20%, about 5% to about 17.5%, about 10% to about 30%, about 10% to about 20%, about 10% to about 17.5%, about 12.5% to about 30%, about 12.5% to about 20%, about 12.5% to about 17.5%, about 15% to about 30%, about 15% to about 20%, or about 15% to about 17.5%, but not limited thereto.

In the case of the method for preparing lysine of the present disclosure, compared to the case where the culture is performed without introduction of carbon dioxide and controlling the concentration of ammonium nitrogen in the medium (maintaining at less than 1.5 g/kg), the produced amount of lysine (concentration of produced lysine) may be increased by about 5% to about 60%, more specifically, about 5% to about 60%, about 5% to about 50%, about 5% to about 45%, about 5% to about 40%, about 5% to about 39.5%, about 15% to about 60%, about 15% to about 50%, about 15% to about 45%, about 15% to about 40%, about 15% to about 39.5%, about 30% to about 60%, about 30% to about 50%, about 30% to about 45%, about 30% to about 40%, about 30% to about 39.5%, about 39% to about 60%, about 39% to about 50%, about 39% to about 45%, about 39% to about 40%, or about 39% to about 39.5%, but not limited thereto.

In the case of the method for preparing lysine of the present disclosure, compared to the case where culture is performed while controlling the concentration of ammonium nitrogen in the medium to be maintained at 1.0 g/kg to 3.0 g/kg through introduction of the nitrogen source as in the present disclosure, but without the carbon dioxide introduction process of the present disclosure, the produced amount of lysine (concentration of produced lysine) may be increased by about 1% to about 20%, more specifically, about 1% to about 20%, about 1% to about 10%, about 1% to about 4%, about 2% to about 20%, about 2% to about 10%, about 2% to about 4%, about 3.5% to about 20%, about 3.5% to about 10%, or about 3.5% to about 4%, but not limited thereto.

In the case of the method for preparing lysine of the present disclosure, compared to the case where the culture is performed by introducing carbon dioxide as in the present disclosure and introducing a nitrogen source according to the present disclosure, but introducing the nitrogen source when the concentration of ammonium nitrogen in the medium becomes less than 0.3 g/kg rather than it becomes less than 1.5 g/kg, the produced amount of lysine (concentration of produced lysine) may be increased by about 10% to about 40%, about 10% to about 30%, about 10% to about 25%, about 15% to about 40%, about 15% to about 30%, about 15% to about 25%, about 20% to about 40%, about 20% to about 30%, or about 20 to 22.5%, but not limited thereto.

In the case of the method for preparing lysine of the present disclosure, compared to the case where the culture is performed without introduction of carbon dioxide and controlling the concentration of ammonium nitrogen in the medium (maintaining at less than 1.5 g/kg), the purity of lysine produced may be increased by about 1% to about 30%, more specifically, about 1% to about 30%, about 1% to about 20%, about 1% to about 14%, about 5% to about 30%, about 5% to about 20%, about 5% to about 14%, about 10% to about 30%, about 10% to about 20%, about 10% to about 14%, about 12.5% to about 30%, about 12.5% to about 20%, or about 12.5% to about 14%, but not limited thereto.

In the case of the method for preparing lysine of the present disclosure, compared to the case where culture is performed while controlling the concentration of ammonium nitrogen in the medium to be maintained at 1.0 g/kg to 3.0 g/kg through introduction of the nitrogen source as in the present disclosure, but without the carbon dioxide introduction process of the present disclosure, the purity of lysine produced may be increased by about 0.1% to about 20%, more specifically, about 0.1% to about 20%, about 0.1% to about 10%, about 0.1% to about 5%, about 0.1% to about 2.5%, about 0.1% to about 1.5%, about 0.5% to about 20%, about 0.5% to about 10%, about 0.5% to about 5%, about 0.5% to about 2.5%, about 0.5% to about 1.5%, about 1.0% to about 20%, about 1.0% to about 10%, about 1.0% to about 5%, about 1.0% to about 2.5%, or about 1.0% to about 1.5%, but not limited thereto.

In the case of the method for preparing lysine of the present disclosure, compared to the case where the culture is performed by introducing carbon dioxide as in the present disclosure and introducing a nitrogen source according to the present disclosure, but introducing the nitrogen source when the concentration of ammonium nitrogen in the medium becomes less than 0.3 g/kg rather than it becomes less than 1.5 g/kg, the purity of lysine produced may be increased by about 1% to about 30%, about 1% to about 25%, about 1% to about 20%, about 5% to about 30%, about 5% to about 25%, about 5% to about 20%, about 10% to about 30%, about 10% to about 25%, about 10% to about 20%, about 12.5% to about 30%, about 12.5% to about 25%, or about 12.5% to about 20%, but not limited thereto.

The term "about" includes all ranges including $\pm0.5$, $\pm0.4$, $\pm0.3$, $\pm0.2$, $\pm0.1$ and the like, and includes all numerical values in an equivalent or similar range to the numerical value following the term about, but not limited thereto.

The method for producing lysine of the present disclosure may further comprise recovering lysine from the medium according to the culture (medium in which culture is performed) or a *Corynebacterium* sp. microorganism. The recovering may be further comprised after the culturing.

The recovering may be collecting targeted amino acids using an appropriate method known in the art depending on the culture method of the microorganism of the present disclosure, for example, batch, continuous or fed-batch culture method and the like. For example, centrifugation, filtration, treatment with a crystallizing protein precipitant (salting out), extraction, ultrasonic disruption, ultrafiltration, dialysis, various kinds of chromatography such as molecular sieve chromatography (gel filtration), adsorption chromatography, ion exchange chromatography, affinity chromatography and the like, HPLC or combinations of these methods may be used, and targeted amino acids may be recovered from the medium or microorganism using a suitable method known in the art.

In addition, the method for producing lysine of the present disclosure, may comprise a purification step additionally. The purification may be performed using an appropriate method known in the art. In one embodiment, when the method for producing L-lysine of the present disclosure comprises both the recovering step and purification step, the recovering step and purification step may be continuously or non-continuously performed regardless of the order, or may be performed simultaneously or by being integrated into one step, but not limited thereto.

In one embodiment, the lysine produced by the method may be obtained in the form of fermented solution comprising lysine. This fermented solution comprising lysine may be further passed through a decarboxylation process and a concentration process, and then, be granulated and dried, and finally, may be obtained as high-content lysine granules.

[ADVANTAGEOUS EFFECTS]

According to the method for preparing lysine according to the present disclosure, by supplying a nitrogen source when the concentration of ammonium nitrogen is less than 1.5g/kg, the ammonium nitrogen in the medium is managed in an optimal level range for lysine biosynthesis to improve the purity of the process solution through reduction of fermentation by-products and improvement of yield, thereby significantly reducing production costs.

[BRIEF DESCRIPTION OF THE DRAWINGS]

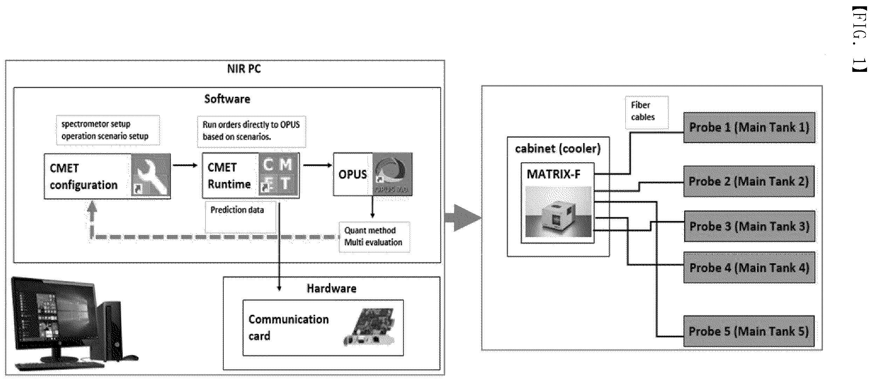

FIG. 1 relates to a process diagram of the real-time monitoring model based on near infrared spectroscopy (NIR spectroscopy) used in the present disclosure.

FIG. 2 is a graph showing the results of cross validation of true measurement values and prediction values of ammonium nitrogen (AN). The x axis represents true AN values (True AN) that spectrum samples collected by NIR equipment are analyzed by Kjeldahl analysis, and the y axis represents prediction AN values (prediction AN) corresponding to true AN values during pretreatment to create a calibration curve.

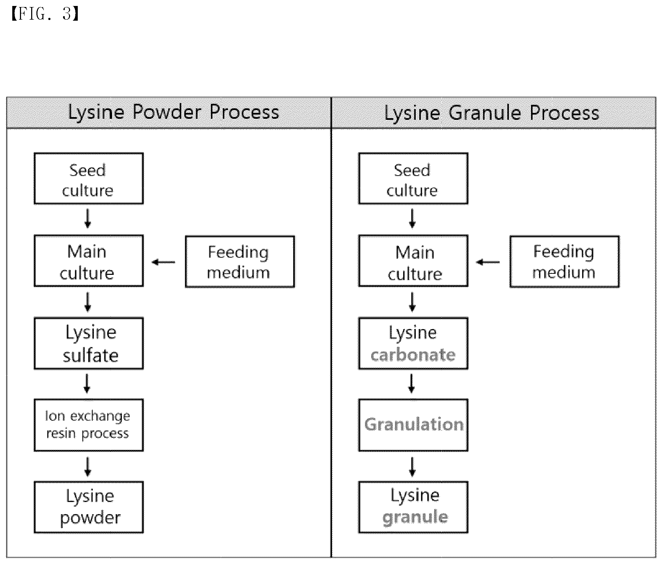

FIG. 3 is a diagram comparing a lysine powder process used conventionally and the lysine granule process used in the present disclosure.

FIG. 4 shows a schematic diagram of the culture process according to a specific example of the present disclosure.

FIG. 5 is a graph confirming the concentration of AN in the medium over time according to the method for preparing lysine according to Example 2, Comparative example 1 and Comparative example 2.

[MODE FOR INVENTION]

Hereinafter, the present disclosure will be described in more detail by examples, but they are merely illustrative and are not intended to limit the scope of the present disclosure. It is obvious to those skilled in the art that the examples described below can be modified without departing from the essential gist of the invention.

[Example]

**Example 1) Method for producing a real-time monitoring model using NIR spectroscopy**

Seed culture of *Corynebacterium glutamicum* CJ3P strain (US 9556463 B2), a Corynebacterium-shaped microorganism having a lysine production ability was performed by solid plate medium and flask culture, and the seed culture step was carried out in a 5L fermenter, and the main culture step was carried out in a 30L fermenter.

The *Corynebacterium glutamicum* CJ3P strain stored in an 80° C cryogenic deep freezer in the form of Glycerol stock (GS) was taken by the 50 $\mu$L level and was inoculated into a solid plate medium produced based on the following medium composition by a streaking method, and cultured in a 30° C incubator for about 24 hours.

To perform flask shaking culture, a liquid medium produced based on the following flask shaking culture medium composition of 100 mL was prepared, and added to a 500 mL shaking flask comprising a baffle, and sterilization was performed using a small autoclave at 121° C for 30 minutes. To conduct shaking flask inoculation, operation was performed on a clean bench. From the cultured solid plate, using a disposable platinum tooth (10 $\mu$L loop), colonies were appropriately taken, and then aseptically inoculated into the shaking culture medium. The inoculated 500 mL shaking flask was cultured at 200 rpm, 30° C for about 10 hours.

Using the seeds obtained from the flask shaking culture, they were inoculated at a ratio of 3.0% (v/v) in a 5 L fermenter comprising a medium in which ammonium sulfate and phosphate were added at a ratio of 0.46 and 0.03 in a molar ratio (target substance mol/glucose mol) based on the glucose used in the seed culture medium composition. The culture was performed under the conditions of pH 6.5-8.5, stirring speed 450 rpm, quantity of airflow 1 vvm in a batch fermentation mode for about 20 hours. The pH was measured with a pH meter (InPro3253i/SG/120, Mettler Toledo) in real time. When the pH was dropped by 0.05 to 0.1 within the range of pH 6.5 to 8.5, the pH was maintained in an appropriate range of pH 6.5 to pH 8.5 by introducing ammonia gas and/or ammonia solution as a nitrogen source through the HMI (Human-Machine Interface) automation program (AVEVA InTouch HMI by AVEVA). Samples were obtained at approximately 2-3 hours intervals, and culture was terminated when residual sugars were depleted.

Using the seeds obtained from the seed culture step, they were inoculated at a ratio of 20.0% (v/v) in a 30 L fermenter comprising a medium in which ammonium sulfate and phosphate were added at a ratio of 0.46 and 0.03 in a molar ratio based on the glucose in the following main culture medium composition. In the main culture step, the culture was performed under the conditions of pH 6.5-8.5, stirring speed 450 rpm, quantity of airflow 1 vvm in a fed-batch fermentation mode. By the method same as above, the pH was measured with a pH meter in real time, and through introducing ammonia gas and/or ammonia solution, the pH was maintained in an appropriate range of pH 6.5 to pH 8.5.

A small amount of broth was obtained from the fermenter through sampling, to check the level of residual sugars using Biochemistry Analyzer YSI2900 analysis equipment, and when the initial carbon source of the medium was depleted, so as to maintain the residual sugars at a level of 0.5 wt% to 1.0 wt%, a feeding medium containing glucose 40.0 wt% was introduced from the feeding tank at a variable flow velocity. Samples were obtained at about 2 hours to 3 hours intervals, and when the ammonium nitrogen concentration (AN: ammonium nitrogen, g/kg) was measured by Kjeldahl analyzer (Foss Kjeltec 8400) and the numerical value was less than 1.5 g/kg, the nitrogen source was constantly maintained through introducing ammonia gas and/or ammonia solution.

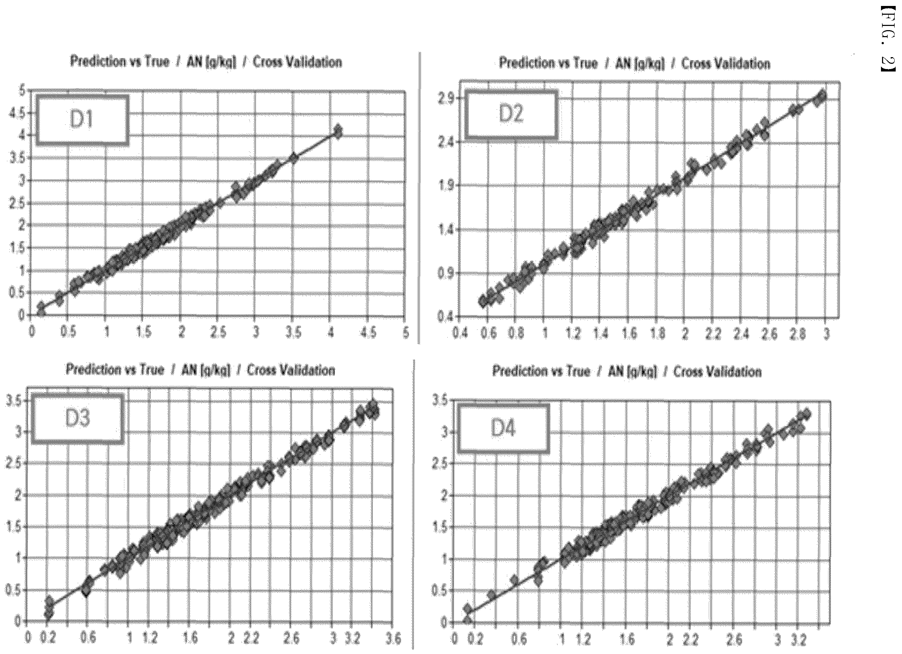

A calibration model was developed using the AN values in fermentation broth samples collected at 2 hours to 3 hours intervals and the corresponding NIR spectral data, utilizing OPUS software (Bruker) obtained by sampling at 2 hours to 3 hours intervals and the NIR spectrum data at the same time was developed. The NIR spectrum was analyzed using Bruker Matrix-F NIR under the wavelength conditions of 3995-11987 cm$^{-1}$, scanner velocity 40 kHz. For the pretreatment technique used to produce the calibration model, among various methods, a vector, a first derivative was selected and used. The probe conditions used for real-time monitoring were resolution 16 cm$^{-1}$, scan time 32 scans, preamplifier Gain A mode, and the cross-validation results that compared the prediction values and true measurement values were as Table 1 and FIG. 2, and a high positive correlation ($R^2$>0.99), RMSECV (Root Mean Square Error of Cross-Validation) 0.06, and RPD (Residual Predictive Deviation) 10.1 numerical values were confirmed.

The calibration curve formula was as follows.

$$y = 0.9854x + 0.0264$$

EP 4 617 369 A1

1) Solid plate medium composition

Glucose 10.0 g/L, bacto tryptone 5.0 g/L, bacto yeast extract 5.0 g/L, NaCl 2.5 g/L, urea 2.0 g/L

2) Flask shaking culture medium composition

Glucose 10.0 g/L. bacto tryptone 5.0 g/L, bacto yeast extract 5.0 g/L, ammonium sulfate 10.0 g/L, urea 2.0 g/L, $KH_2PO_4$ 5.0 g/L, $K_2HPO_4$ 10.0 g/L, $MgSO_4$ $7H_2O$ 0.5 g/L

3) Fermenter (5 L) seed culture medium composition

Antifoaming agent 1 mL/L, corn steep liquor 10.0 g/L, biotin 1.0 mg/L, thiamine 10.0 mg/L, pantothenic acid 10.0 mg/L, niacinamide 10.0 mg/L

4) Fermenter (30 L) main culture medium composition

Antifoaming agent 1 mL/L, corn steep liquor 10.0 g/L, biotin 1.0 mg/L, thiamine 10.0 mg/L, pantothenic acid 10.0 mg/L, niacinamide 10.0 mg/L

[Table 1]

| Item | Fermenter 1(D1) | Fermenter 2(D2) | Fermenter 3(D3) | Fermenter 4(D4) | Mean (n=4) |
|---|---|---|---|---|---|
| $R^2$ | 99.1 | 99.2 | 99.0 | 98.6 | 99.0 |
| RMSECV | 0.06 | 0.05 | 0.07 | 0.07 | 0.06 |
| RPD | 10.8 | 11.1 | 10.0 | 8.4 | 10.1 |

As above, a model capable of monitoring AN value in real time with high accuracy could be established.

**Example 2) Method for preparing lysine using carbon dioxide gas automated introduction technology**

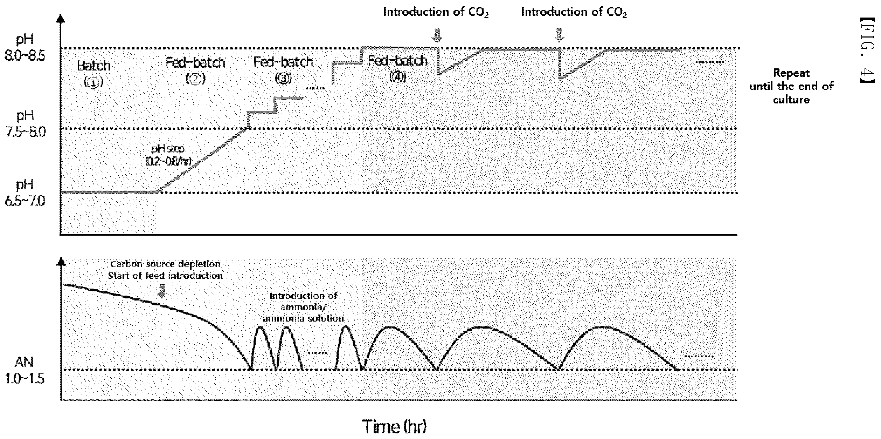

Using the seeds obtained by the same method as the seed culture step of Example 1, they were inoculated at a ratio of 20.0% (v/v) in a 30 L fermenter comprising a medium in which ammonium sulfate and phosphate were added at a ratio of 0.46 and 0.03 in a molar ratio based on the glucose in the main culture medium composition. In the main culture, the culture was performed under the conditions of pH 6.5 to pH 8.5, stirring speed 450 rpm, and quantity of air flow 1 vvm in a fed-batch fermentation mode. The pH was measured with a pH meter (InPro3253i/SG/120, Mettler Toledo) in real time. The main culture was performed as follows, and the schematic diagram of the culture process was shown in FIG. 4.

1) In a batch fermentation mode, in order to maintain a pH close to neutrality within the optimal pH for culture from the time of the start of culture until the depletion of the initial carbon source of the medium, the pH set-point was set to pH 6.5 or more and less than pH 7.0, and to maintain the pH of the medium at the set-point, ammonia gas and/or ammonia solution were introduced as nitrogen sources by HMI (Human-Machine Interface) automated program (AVEVA InTouch HMI by AVEVA). At this time, since the consumption amount of the nitrogen sources was greater than the supply amount thereof, the AN value gradually decreased (section ① of FIG. 4).

2) After the initial carbon source was depleted, by the same method as Example 1, a feeding medium containing glucose 40.0 wt% was added to maintain residual sugars at a level of 0.5 wt% to 1.0wt%, and the process was carried out in a fed-batch fermentation mode. After adding the feeding medium, in order to increase the ammonia supply amount sufficient for lysine biosynthesis, through the HMI automated program, the pH set-point was raised at a rate of 0.2-0.8/hr until it reached pH 7.5 or more and less than pH 8.0,and so as to maintain the medium pH at the set-point, ammonia gas and/or ammonia solution as a nitrogen source was continuously introduced, thereby keeping the AN value within the optimal range for lysine biosynthesis, from 1.0 g/kg to 3.0 g/kg. At this time, since the ammonia supply amount and consumption amount were similar, the AN gently decreased within the AN optimal range (section ② of FIG. 4).

3) From the time when the pH of the medium first reached pH 7.5 or more and less than pH 8.0, for more detailed culture condition management, the following process was performed using the AN real-time monitoring method using NIR spectroscopy established in Example 1. When the pH of the medium was pH 7.5 or more and less than pH 8.0, and the AN value was lowered to less than 1.5 g/kg, the set-point of the fermentation pH was slightly increased (by approximately 0.05), and to reach the set-point, nitrogen sources, ammonia gas and/or ammonia solution, were automatically introduced to maintain the AN value in the range of 1.0 g/kg to 3.0 g/kg (section ③ of FIG. 4). The above process was repeated to maintain the AN value in the range of 1.0 g/kg to 3.0 g/kg. However, since the growth rate and metabolic efficiency of *Corynebacterium* sp. microorganisms may decrease when the fermentation pH exceeds the range of pH 8.0 or more and less than pH 8.5, it was repeated only until the fermentation pH reached pH 8.0 or more and less than pH 8.5. Ammonia gas and/or ammonia solution was introduced through the HMI automated program until the present value of pH reached the pH set-point by controlling the rate with on/off time (1 sec/10 sec).

4) From the time when the pH of the medium first reached pH 8.0 or more and less than pH 8.5, carbon dioxide gas was automatically introduced to lower the pH, when the pH of the medium was pH 8.0 or more and less than pH 8.5 and the AN value in the medium became less than 1.5 g/kg due to lack of nitrogen source according to lysine biosynthesis, using the AN real-time monitoring method using NIR spectroscopy established in Example 1. Specifically, through the HMI automated program, carbon dioxide gas was introduced at about 1.5 vvm (gas introduction amount L / medium volume L / minute), to lower the pH of the medium by 0.1 to 0.4 (about 0.2). When the pH decreased in this manner, ammonia gas and/or ammonia solution as a nitrogen source was automatically introduced so as to allow the pH of the medium to reach the final pH set-point set previously in process 3), ensuring that the AN value was maintained within the range of 1.0 g/kg to 3.0 g/kg. After the above process was performed, when the pH of the medium again reached pH 8.0 or more and less than pH 8.5 and the AN value again became less than 1.5 g/kg due to nitrogen source depletion caused by lysine biosynthesis, the process of introducing carbon dioxide gas to lower the pH and introducing ammonia gas and/or ammonia solution to restore the pH to the set-point previously set, was carried out as described earlier. In this manner, the above process was repeatedly performed until the end of the culture to maintain the AN in the range of 1.0 g/kg to 3.0 g/kg (section ④ of FIG. 4).

At the end of the culture, remaining sugars were not present, and the fermentation time was 30.3 hours. During fermentation, the maximum pH that could be increased was less than pH 8.5 due to the automated carbon dioxide introduction system. Compared to the result of Comparative example 2 described later, improvement of the lysine concentration by 4.0% and reduction of the concentration of the by-products by 28.6% were confirmed, and therefore, it was confirmed that the normalized purity obtained from the fermentation process was improved by 1.3%.

**Comparative example 1) Method for preparing lysine with AN of less than 1.5 g/kg**

Using the seeds obtained by the same method as the seed culture step of Example 1, they were inoculated at a ratio of 20.0% (v/v) into a 30 L fermenter comprising a medium in which ammonium sulfate and phosphate were added at a ratio of 0.46 and 0.03 in a molar ratio based on the glucose in the main culture medium composition. In the main culture step, they were cultured under the conditions of stirring speed 450 rpm and quantity of airflow 1 vvm in a fed-batch fermentation mode. When the initial carbon source of the medium was depleted, a feeding medium comprising glucose 40.0 wt% was added by the same method as Example 1 to maintain the residual sugars at a level of 0.5-1.0wt%.

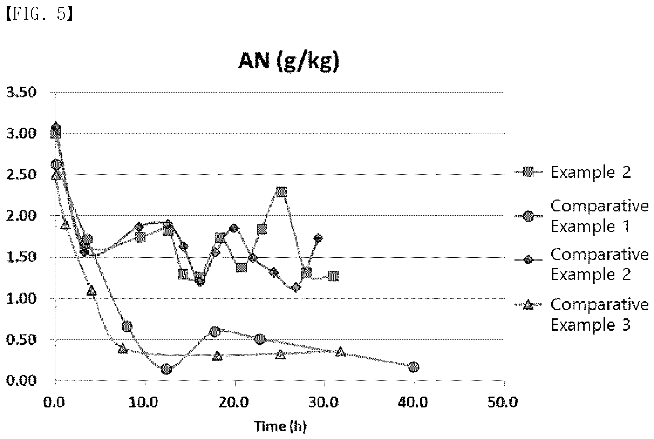

At the end of the culture, the residual sugars were not present, but as FIG. 5, due to absence of management of the AN value, the AN value was maintained less than 1.5 g/kg. The fermentation time was 39.9 hours due to delay, and compared to the result of Comparative example 2 described later, it could be confirmed that the lysine concentration was reduced by 25.4% and the normalized purity was reduced by 10.4% and therefore, they were significantly dropped.

**Comparative example 2) Method for preparing lysine using only ammonia without introduction of carbon dioxide**

Using the seeds obtained by the same method as the seed culture step of Example 1, they were inoculated at a ratio of 20.0% (v/v) into a 30 L fermenter comprising a medium in which ammonium sulfate and phosphate were added at a ratio of 0.46 and 0.03 in a molar ratio based on the glucose in the main culture medium composition. In the main culture step, they were cultured under the conditions of pH 6.5-8.5, stirring speed 450 rpm and quantity of airflow 1 vvm in a fed-batch fermentation mode. When the pH was dropped by 0.05 to 0.1 within the range of pH 6.5 to 8.5, the pH was maintained in an appropriate range of pH 6.5 to pH 8.5 by introducing ammonia gas and/or ammonia solution as a nitrogen source through the HMI (Human-Machine Interface) automation program (AVEVA InTouch HMI by AVEVA).

In the present Comparative example, the processes of 1) to 3) of Example 2 were performed as described, but the process 4) of Example 2, which involving lowering the pH through automatic carbon dioxide gas introduction when the fermentation pH reached the upper limit (pH 8.0 or more and less than pH 8.5) and the AN value became less than 1.5 g/kg, was not performed. As a result, the pH of the medium could be increased to pH 8.5 or more.

At the end of the culture, the residual sugars were not present, and the fermentation time was 28.7 hours, and the lysine concentration was at a level of 192.4 g/L.

**Comparative example 3) Method for preparing lysine at an AN level of less than 0.5 g/kg using automated carbon dioxide gas introduction system**

Using the seeds obtained by the same method as the seed culture step of Example 1, they were inoculated at a ratio of 20.0% (v/v) into a 30 L fermenter comprising a medium in which ammonium sulfate and phosphate were added at a ratio of 0.46 and 0.03 in a molar ratio based on the glucose in the main culture medium composition. In the main culture step, they were cultured under the conditions of pH 6.5-8.5, stirring speed 450 rpm and quantity of airflow 1 vvm in a fed-batch fermentation mode.

In the present Comparative example, the processes of 1) to 3) of Example 2 were performed as described. However, whereas in process 4) of Example 2, the pH was lowered through automatic carbon dioxide gas introduction when the fermentation pH reached the upper limit (pH 8.0 or more and less than pH 8.5) and the AN value became less than 1.5 g/kg, in this comparative example, carbon dioxide gas was automatically introduced to decrease the pH of the medium by 0.1 to 0.4 (approximately 0.2) when the fermentation pH reached the upper limit (pH 8.0 or more and less than pH 8.5) and the AN value became less than 0.3 g/kg. Additionally, ammonia gas and/or an ammonia solution as a nitrogen source was automatically introduced to maintain the AN value within a range of less than 0.5 g/kg.

As the supply of AN, an essential nitrogen source for lysine biosynthesis, was limited to a level of less than 0.5 g/kg, the lysine concentration was 164.1 g/L, representing an 18.0% decrease compared to the results of Example 2 described above. Additionally, with a 660.0% increase in by-products, the standardized purity significantly decreased to 13.3%. Furthermore, as fermentation progressed, the sugar consumption rate declined sharply, resulting in a fermentation time of 31.7 hours, with approximately 4.8 g/L of residual sugar remaining at the end of the culture.

The culture results of Example 2, Comparative example 1 and Comparative example 2 are shown in Table 2 below for comparison, while the culture results of Example 2 and Comparative example 3 are shown in Table 3 below for comparison. The AN concentration in the medium thereof was shown in FIG. 5.

[Table 2]

| Item | Unit | Example 2 | Comparative example 1 | Comparative example 2 |
|---|---|---|---|---|
| Fermentation time | Hr | 30.3 | 39.9 | 28.7 |
| Residual sugars | g/L | 0.0 | 0.0 | 0.0 |
| By-products* | % | 71.4 | - | 100.0 |
| Lysine | g/L | 200.0 | 143.5 | 192.4 |
| Purity* | % | 101.3 | 89.6 | 100.0 |
| * Normalization result values based on 100% of the result of | | | | |

Comparative example 2

[Table 3]

| Item | Unit | Example 2 | Comparative example 3 |
|---|---|---|---|
| Fermentation time | Hr | 30.3 | 31.7 |
| Residual sugars | g/L | 0.0 | 4.8 |
| By-products* | % | 100.0 | 660.0 |
| Lysine | g/L | 200.0 | 164.1 |
| Purity* | % | 100.0 | 86.7 |
| * Normalization result values based on 100% of the result of Example 2 | | | |

In summary of the experimental results of Tables 2 and 3, Example 2 used the real-time AN monitoring method established in Example 1 in the main culture step. When the concentration of AN became less than 1.5 g/kg during fermentation, the fermentation pH was gradually increased to maximum range of pH 8.0 or more and less than pH 8.5 in order to supply the nitrogen source. Once the pH of the medium reached to pH 8.0 or more and less than pH 8.5, carbon dioxide gas and ammonia gas and/or ammonia solution were introduced to maintain the fermentation pH to pH 7.5 or more and less than pH 8.5. Therefore, compared to the result of Comparative example 2, reduction in normalized by-products by 28.6% and improvement of lysine by 4.0% were observed under equivalent fermentation durations, resulting in a 1.3% improvement in the final normalized process solution purity.

Comparative example 1 conducted lysine fermentation without the process of managing AN according to the real-time AN monitoring method established in Example 1 in the main culture step, and the AN values were not managed, so the AN was maintained less than 1.5 g/kg for most of the period of fermentation conduced in a fed-batch mode. Due to absence of management of AN, the most important factor in lysine production, it was observed that the fermentation time was delayed by about 39.0% and lysine production was decreased by 25.4%, compared to Comparative example 2.

In Comparative Example 3, the real-time AN monitoring method established in Example 1 was applied during the main culture step. When the AN level became less than 0.3 g/kg during fermentation, the fermentation pH was gradually increased to a maximum range of pH 8.0 or more and less than pH 8.5 to supply the nitrogen source. Once the pH reached this range, carbon dioxide gas, ammonia gas, and/or ammonia solution were introduced to maintain the fermentation pH within a range of pH 7.5 or more and less than pH 8.5. Therefore, compared to the results of Example 2, the lysine concentration was 164.1 g/L due to the limitation of the maximum AN level to 0.5 g/kg, representing an 18.0% decrease compared to the results of Example 2 described above. Additionally, with a 660.0% increase in by-products, the standardized purity significantly decreased by 13.3%, confirming a substantial reduction. Furthermore, as fermentation progressed, the sugar consumption rate declined sharply, resulting in a fermentation time of 31.7 hours, with approximately 4.8 g/L of residual sugar remaining at the end of the cultivation.

These results indicate that maintaining the AN level below 0.5 g/kg during fermentation negatively affects fermentation indicators. It was observed that when the AN level drops below 1.5 g/kg, the addition of a nitrogen source and/or carbon dioxide to the medium to maintain an appropriate range of AN between 1.0 g/kg and less than 3.0 g/kg is essential for lysine production.

The ion exchange resin process significantly increases production costs due to addition of a purification process and introduction of subsidiary raw materials, and in a process of preparing granules using lysine carbonate, in order to improve the purity of a final product, it is necessary to reduce by-products in the obtained fermented solution and improve the concentration. In a lysine granule process without a separate ion exchange resin process, a problem of dropping the purity of a product derived from the fermented solution by-products occurs, but when the carbon dioxide fermentation method in which the present technology is introduced is used, during culture, the concentration of the by-products is reduced and the concentration of lysine is significantly improved, and therefore, the fermentation purity can be improved and the granule product quality can be enhanced.

**Claims**

1. A method for preparing lysine by a fermentation process using a microorganism having an ability to produce lysine, **characterized by**

   monitoring the concentration of ammonium nitrogen in a medium, and
   introducing a nitrogen source into the medium, when the concentration of ammonium nitrogen in the medium is less than 1.5 g/kg.

2. The method for preparing lysine according to claim 1, wherein the concentration of ammonium nitrogen in the medium is controlled to be maintained at 1.0 g/kg to 3.0 g/kg.

3. The method for preparing lysine according to claim 1, wherein the nitrogen source is at least one selected from the group consisting of ammonia gas and ammonia solution.

4. The method for preparing lysine according to claim 1, wherein the pH of the medium is further monitored, and

   when the pH of the medium is pH 7.5 or more and less than pH 8.0 and the concentration of ammonium nitrogen in the medium is less than 1.5 g/kg,
   the pH set-point is increased, and
   the nitrogen source is introduced to the medium so that the pH of the medium can reach the pH set-point.

5. The method for preparing lysine according to claim 1, wherein the pH of the medium is additionally monitored, and

   when the pH of the medium is pH 8.0 or more and less than pH 8.5 and the concentration of ammonium nitrogen in the medium is less than 1.5 g/kg,

carbon dioxide is introduced into the medium.

6. The method for preparing lysine according to claim 5, wherein the carbon dioxide is introduced until the pH of the medium decreases by 0.1 to 0.4.

7. The method for preparing lysine according to claim 5, wherein the carbon dioxide is introduced at 0.5 vvm to 3.0 vvm.

8. The method for preparing lysine according to claim 5, wherein the nitrogen source is introduced into the medium so that the pH of the medium decreased by introduction of carbon dioxide can be increased.

9. The method for preparing lysine according to claim 8, wherein the nitrogen source is introduced simultaneously or sequentially with introducing the carbon dioxide to the medium.

10. The method for preparing lysine according to claim 5, wherein the pH of the medium is increased from pH 6.5 or more and less than pH 7.0 to pH 7.5 or more and less than pH 8.5, and then carbon dioxide is introduced to control the pH of the medium to be maintained at pH 7.5 or more and less than pH 8.5.

11. The method for preparing lysine according to claim 1, wherein the monitoring the concentration of ammonium nitrogen in the medium is performed using a near infrared (NIR) spectrometric analyzer.

12. The method for preparing lysine according to claim 1, wherein the medium comprises ammonium sulfate as a nitrogen source.

13. The method for preparing lysine according to claim 1, wherein the ammonium sulfate is not further added into the medium during the fermentation process.

【FIG. 1】

【FIG. 2】

【FIG. 3】

| Lysine Powder Process | Lysine Granule Process |
|---|---|

Seed culture → Main culture ← Feeding medium → Lysine sulfate → Ion exchange resin process → Lysine powder

Seed culture → Main culture ← Feeding medium → Lysine carbonate → Granulation → Lysine granule

【FIG. 4】

【FIG. 5】

## INTERNATIONAL SEARCH REPORT

International application No.

**PCT/KR2025/001580**

| A. | CLASSIFICATION OF SUBJECT MATTER |
|---|---|

**C12P 13/08**(2006.01)i; **C12N 1/38**(2006.01)i

According to International Patent Classification (IPC) or to both national classification and IPC

| B. | FIELDS SEARCHED |
|---|---|

Minimum documentation searched (classification system followed by classification symbols)

C12P 13/08(2006.01); A23K 10/12(2016.01); A23K 20/142(2016.01); C12M 1/34(2006.01); C12M 1/36(2006.01); C12N 1/00(2006.01); C12N 1/18(2006.01); C12P 1/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 라이신(lysine), 암모늄 질소(ammonium nitrogen), 황산 암모늄(ammonium sulfate), 코리네박테리움(corynebacterium), 이산화탄소(carbon dioxide), 발효(fermentation)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | US 2018-0116246 A1 (CJ DO BRASIL INDUSTRIA E COMERCIO DE PRODUTOS ALIMENTICIOS LTDA.) 03 May 2018 (2018-05-03)<br>See claim 1; and paragraph [0047]. | 1-13 |
| A | KR 10-2007-0061587 A (AJINOMOTO CO., INC.) 13 June 2007 (2007-06-13)<br>See claims 1-5. | 1-13 |
| A | JP 10-210995 A (CHISSO CORP.) 11 August 1998 (1998-08-11)<br>See claims 1-13. | 1-13 |
| A | KR 10-2016-0062151 A (AJINOMOTO CO., INC.) 01 June 2016 (2016-06-01)<br>See claims 1-27. | 1-13 |
| A | CN 116732116 A (QIQIHAR LONGJIANG FUFENG BIOTECHNOLOGY CO., LTD.) 12 September 2023 (2023-09-12)<br>See claims 1-3. | 1-13 |

☐ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **02 May 2025** | **02 May 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2025/001580**

| Patent document cited in search report | | | Publication date (day/month/year) | Patent family member(s) | | | Publication date (day/month/year) |
|---|---|---|---|---|---|---|---|
| US | 2018-0116246 | A1 | 03 May 2018 | CN | 107872957 | A | 03 April 2018 |
| | | | | EP | 3283636 | A1 | 21 February 2018 |
| | | | | WO | 2016-164996 | A1 | 20 October 2016 |
| KR | 10-2007-0061587 | A | 13 June 2007 | CN | 101111602 | A | 23 January 2008 |
| | | | | CN | 101111602 | B | 04 July 2012 |
| | | | | CN | 103088080 | A | 08 May 2013 |
| | | | | CN | 103088080 | B | 17 February 2016 |
| | | | | EP | 1813677 | A1 | 01 August 2007 |
| | | | | EP | 1813677 | A4 | 19 October 2011 |
| | | | | EP | 1813677 | B1 | 06 February 2019 |
| | | | | EP | 2818554 | A2 | 31 December 2014 |
| | | | | EP | 2818554 | A3 | 11 February 2015 |
| | | | | EP | 2818555 | A2 | 31 December 2014 |
| | | | | EP | 2818555 | A3 | 18 February 2015 |
| | | | | EP | 2818555 | B1 | 28 December 2022 |
| | | | | EP | 2818556 | A2 | 31 December 2014 |
| | | | | EP | 2818556 | A3 | 18 February 2015 |
| | | | | EP | 2818556 | B1 | 10 May 2023 |
| | | | | JP | 2008-038695 | A1 | 15 May 2008 |
| | | | | JP | 2008-038695 | A5 | 02 October 2008 |
| | | | | JP | 4844396 | B2 | 28 December 2011 |
| | | | | KR | 10-0921644 | B1 | 14 October 2009 |
| | | | | KR | 10-2009-0023513 | A | 04 March 2009 |
| | | | | US | 2007-0243590 | A1 | 18 October 2007 |
| | | | | US | 2010-0273221 | A1 | 28 October 2010 |
| | | | | US | 2012-0149072 | A1 | 14 June 2012 |
| | | | | US | 2015-0010962 | A1 | 08 January 2015 |
| | | | | US | 2015-0010963 | A1 | 08 January 2015 |
| | | | | US | 2015-0259716 | A1 | 17 September 2015 |
| | | | | US | 7790422 | B2 | 07 September 2010 |
| | | | | US | 8198053 | B2 | 12 June 2012 |
| | | | | US | 8859242 | B2 | 14 October 2014 |
| | | | | US | 9062335 | B2 | 23 June 2015 |
| | | | | US | 9334511 | B2 | 10 May 2016 |
| | | | | US | 9334512 | B2 | 10 May 2016 |
| | | | | WO | 2006-038695 | A1 | 13 April 2006 |
| JP | 10-210995 | A | 11 August 1998 | JP | 3944934 | B2 | 18 July 2007 |
| KR | 10-2016-0062151 | A | 01 June 2016 | CN | 106459857 | A | 22 February 2017 |
| | | | | CN | 106459857 | B | 19 April 2019 |
| | | | | EP | 3053999 | A1 | 10 August 2016 |
| | | | | EP | 3053999 | A4 | 30 November 2016 |
| | | | | EP | 3053999 | B1 | 20 November 2019 |
| | | | | JP | 2016-106637 | A | 20 June 2016 |
| | | | | JP | 2017-050234 | A1 | 09 March 2017 |
| | | | | JP | 5958653 | B2 | 02 August 2016 |
| | | | | KR | 10-1783681 | B1 | 10 October 2017 |
| | | | | US | 2015-0337254 | A1 | 26 November 2015 |
| | | | | US | 9708577 | B2 | 18 July 2017 |
| | | | | WO | 2015-050234 | A1 | 09 April 2015 |
| CN | 116732116 | A | 12 September 2023 | None | | | |

Form PCT/ISA/210 (patent family annex) (July 2022)

22

**INTERNATIONAL SEARCH REPORT**
Information on patent family members

| International application No. |
| --- |
| **PCT/KR2025/001580** |

| Patent document cited in search report | Publication date (day/month/year) | Patent family member(s) | Publication date (day/month/year) |
| --- | --- | --- | --- |
| | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240016196
- CN 110484575
- KR 100838200
- US 9556463 B2